# EUROPEAN PATENT SPECIFICATION

(11) **EP 1 194 124 B1**
(45) Date of publication and mention of the grant of the patent: **03.09.2003**
(21) Application number: 00931481.6
(22) Date of filing: 07.06.2000
(51) Int. Cl.: A61K 9/16, A61K 9/22, A61K 47/30

(54) **TASTE MASKED COMPOSITIONS**
GESCHMACKSMASKIERTE ZUBEREITUNGEN
COMPOSITIONS A GOUT MASQUE

(30) Priority: 11.06.1999 IN DE086799; 05.06.2000 US 587535
(43) Date of publication of application: 10.04.2002
(73) Proprietor: Ranbaxy Laboratories Limited, New Delhi 110 019 (IN)
(72) Inventor: MUKHERJI, Gour, Gurgaon 122 001, Haryana (IN); GOEL, Sandhya, Delhi 110 034 (IN); ARORA, Vinod, Kumar, Vikas Puri, New Delhi 110 018 (IN)
(74) Representative: Cronin, Brian Harold John
(86) International application number: IB0000765
(87) International publication number: WO00076479

(56) References cited:
- WO-A1-97/16174
- WO-A1-98/11879
- WO-A1-98/18454
- DE-A- 2 218 147
- US-A- 4 897 270
- US-A- 5 175 003
- VOIGT R.: "Pharmazeutische Technologie f r Studium und Beruf" 1993, ULLSTEIN MOSBY, BERLIN, DE, XP002901233 7th edition, chapter 10.5.1, table 29, chapter 10.6.2.2.- -10.6.2.4.

## Description

The present invention relates to taste masked compositions for bitter drugs, comprising a combination of two enteric polymers, such as a methacrylic acid copolymer, and a phthalate polymer. It also relates to a process for preparing such a composition.

### BACKGROUND OF THE INVENTION

For ease and safety of administration, most drugs are formulated as tablets or capsules for oral administration. However, patients at the extremes of age, such as children and the elderly, often experience difficulty in swallowing solid oral dosage forms. For these patients, drugs are commonly provided in liquid dosage forms such as solutions, emulsions and suspensions. These dosage forms usually permit perceptible exposure of the active drug ingredient to the taste buds, which can be a problem when the drugs have an unpleasant taste or are extremely bitter. Conventional taste masking techniques such as the use of sweeteners, amino acids and flavoring agents are often unsuccessful in masking the taste of highly bitter drugs and other techniques have been and continue to be exploited for the effective taste masking of such drugs. Extremely bitter drugs, like, quinine, ciprofloxacin, clarithromycin, cefuroxime axetil, can now be formulated as a fairly acceptable range of products even for pediatric use, which through conventional techniques would be impossible to formulate.

Use of cation - exchange resins (such as polysulfonic acid and polycarboxylic acid polymers) to adsorb amine drugs for taste masking and sustained release has been reported to have limited applicability and is not capable of masking the taste of highly bitter drugs. Coating of bitter drugs is another method which has been reported for taste masking. This technique alone may prove effective for moderately bitter drugs or in products where the coated particles are formulated as aqueous preparations before administration or are formulated in a non-aqueous medium. This technique has its limitations as coating of fine particles is usually technology intensive and coated granules are readily ruptured by chewing and compression.

Lipid-based microencapsulation is another technique used to taste mask the drugs. This technique requires highly sophisticated hot-melt granulation for producing fine particles, and may have adverse effects on heat sensitive molecules or restrict drug release adversely. U.S. Patent No. 4,865,851 describes cefuroxime axetil in particulate form coated with an integral coating of lipid or a mixture of lipids.

U.S. Patent No. 4,808,411 describes a taste-masked composition comprising 95% of erythromycin or a derivative thereof and about 5 to about 75% of a carbomer. The drug and carbomer are believed to be held together by both the ionic interactions between the amine group of erythromycin compound and the carbonyl group of the carbomer and by the gel properties of the carbomer. This complex is further taste masked by coating. Although use of this complexing technique, optionally with a coating, has evolved into a useful technique for taste-masking, proper selection of the complexing agent is vital such that in trying to achieve taste-masking, drug release is not compromised.

U.S. Patent No. 5,286,489 describes a porous drug-polymer matrix formed by admixing one or more bitter tasting active ingredient and a methyl methacrylic ester copolymer in at least a 1:1. by weight ratio of active ingredlent to copolymer, effective to mask the taste of the drug. None of the examples described in this patent disclose the effect of these polymers on the release of the drug from the matrix. It has been our experience that although the drug-polymer matrix formed following the teachings of this patent results in good taste-masking, it also retards the rate of drug release from the matrix to an extent which would be unacceptable for a conventional immediate release formulation. Following the teachings of this patent, only 42% of cefuroxime axetil was released from the matrix in 45 minutes in media of pH greater than 4.0. The matrix described in this patent is therefore unsuitable for drugs which are absorbed at a pH range greater than 4.0. To enhance the release of the drug, an enteric phthalate polymer was added Into the matrix without significantly compromising on the taste masking.

WO 98/18454 discloses an enteric coating composition comprising a blend of an alkali-soluble acrylic latex polymer and an aqueous solution of ammonium or alkaline salts of cellulose polymers. However this reference discloses a coating and not a polymeric matrix as claimed herein.

Accordingly, none of the references heretofore described is completely satisfactory for various reasons.

### SUMMARY OF THE INVENTION

The object of the present invention is to provide a taste masked composition, which effectively masks the taste of the drug without compromising the dissolution rate, comprising a bitter tasting drug and a combination of two enteric polymers comprising a methacrylic acid copolymer and a phthalate polymer.

A further object of the present invention is to describe a process for the preparation of a taste masked matrix comprising the process of dissolving the bitter tasting drug, a methacrylic acid copolymer and a phthalate polymer, in a suitable organic solvent followed by the recovery of said taste masked matrix from the solution thereof.

### DETAILED DESCRIPTION OF THE INVENTION

The taste masked composition of the present invention comprises a bitter tasting active, and two enteric polymers wherein the enteric polymers are a methacrylic acid copolymer and a phthalate polymer. Examples of bitter or unpleasant tasting drugs which may be used, include, but are not limited to, macrolide antibiotics, such as erythromycin and clarithromycin, fluoroquinolones such as ciprofloxacin and norfloxacin, cephalosporins such as cefuroxime and ceftriaxone, tetracyclic antibiotics like chloramphenicol, chlorpromazine etc. The drug itself or its pharmaceutically acceptable salt or ester may be used in the present invention.

The methacrylic acid copolymers used according to the present invention, may include methylmethacrylic ester copolymers, such as Eudragit™ S and Eudragit™ L (trademark of Rohm Pharma) and copolymers of ethyl acrylate and methacrylic acid as Eudragit™ L-100-55 (trademark of Rohm Pharma). The phthalate polymers include cellulose acetate phthalate, ethyl vinyl phthalate, polyvinyl acetate phthalate and hydroxy alkyl cellulose phthalates. This combination of the two enteric polymers, methacrylic acid copolymer and a phthalate polymer results in optimal taste-masking and dissolution characteristics of the drug. The ratio of methacrylic acid copolymer to phthalate polymer can be varied from 1:9 to 9:1 depending upon intensity of bitterness and desired release of the active ingredient. Most preferably the two polymers are in the ratio of 1:1.

Preferably for optimal taste masking effect, total polymer to drug ratio is at least 1:4.

According to the present invention, the taste masked matrix described above, is prepared by dissolving, optionally with heating, the bitter active ingredient, a methacrylic acid copolymer and a phthalate polymer in a solvent system and then recovering the matrix including the active ingredient and the two polymers from the solution thereof. The solvent system chosen is one in which, both the active ingredient and the polymers are either soluble or swellable. Preferred solvents Include water, ketones such as acetone, alcohols such as ethanol, esters such as ethyl acetate and their mixtures. The matrix is recovered by conventional methods which include vacuum evaporation, tray drying, spray drying, and drum or belt film drying. Spray drying is the preferred method for solvent removal. The "solid solutions" thus formed keep the drug in a finely dispersed state within the polymers, preventing the exposure of the bitter tasting drug to the taste buds.

The process of spray drying gives highly porous material which can be further compacted to granules to improve the taste masking effect. The porosity of the granules thus obtained, is not only important for dissolution but also determines the extent of taste masking.

Channeling agents can be used to further tailor the drug release from the compacted granules. Channeling agents help in opening up the granules in a specific media as desired. The channeling agents include, disintegrants like croscarmellose sodium, crospovidone and sodium starch glycolate, diluents such as lactose, mannitol, sodium chloride, talc, polyvinyl pyrrolidone; gelling agents like carbopol, and xanthan gum, among others.

The taste masked granules obtained may be mixed with flavoring agents such as natural or artificial flavors, citric and tartaric acids, sweeteners such as saccharin and aspartame, and with other pharmaceutically acceptable excipients to be formulated as conventional whole, chewable or dispersible tablets, dry syrups, suspensions, sachets or any other suitable oral dosage forms.

The use of particle coating offers additional taste masking on the product. The coating composition can be constituted of either pH dependent or pH independent polymers depending on desired product characteristics. Coating compositions such as that described in our PCT application WO 00/56266 offer additional advantages as they can effectively mask the remaining bitterness of the drug without significantly affecting Its dissolution profile. Other coating polymers such as the cellulosic polymers and methacrylic acid copolymers may also be used to optimize the taste masking effect.

The examples given herein further illustrate the effectiveness of our formulation in achieving both taste-masking and optimal dissolution of the drug from the matrix.

### Example 1

2 g of cefuroxime axetil was taken together with 2 g polymer mixture (0.7: 0.3, Eudragit™ L100-55: hydroxypropyl methyl cellulose phthalate) and dissolved in acetone (20 ml) containing 5% water. The resulting mixture was tray dried and sized to obtain BSS mesh 44/85 particles. These granules showed adequate taste masking and 95% of the drug was released from the matrix within 45 minutes in pH 6.8 phosphate buffer.

### Example 2

20 g cefuroxime axetil and 40 g total polymer (1.2:0.8 w/w mixture of Eudragit™ L-100-55 and hydroxypropyl methyl cellulose phthalate) were dissolved in 112ml acetone and 16 ml water mixture. The solvent was removed by tray drying under vacuum at 40°C for 12 hours. The dried mass was milled to get a BSS mesh 44/85 size fraction. The granules thus obtained showed adequate taste masking and released 100% of the drug in 45 minutes in pH 6.8 phosphate buffer.

### Example 3

60 g cefuroxime axetil and 60 g total polymer (1:1 w/w mixture of Eudragit™ L-100-55 and hydroxypropyl methyl cellulose phthalate) were dissolved under stirring in a mixture of 875 ml acetone and 125 ml water at 35-40°C. The solvent was removed by spray drying. The spray dried material was further dried for 12 hours at 40°C under vacuum to obtain a fluffy and amorphous material. This spray dried material was compacted and milled to obtain granules of the desired particle size and taste (BSS mesh 44/85). The granules thus obtained released 100% of the drug from the matrix within 45 minutes in pH 6.8 buffer, These granules therefore showed ideal characteristics of both taste masking and drug release from the matrix.

### Example 4

75 g clarithromycin and 75 g polymer (1:1 mixture of hydroxypropyl methyl cellulose phthalate + Eudragit™ L-100-55) were dissolved under stirring in a mixture of acetone (110ml) and water (15ml) at 45-50°C. The solvent was removed on Buchi™ rotavapor and the thick viscous mass so obtained was tray dried at 60°C to obtain a flaky, partially taste masked material. The product thus obtained was milled to give partially taste masked granules of the desired particle size (BSS mesh 44/85).

These granules were further coated with the coating composition described in our PCT application WO 00/56266 to yield a bitterless material suitable for use in an oral suspension. These coated granules released 84% of the drug within 60 minutes in pH 6.8 phosphate buffer.

## Claims

1. A taste masked matrix composition comprising a bitter tasting drug, and a combination of two enteric polymers comprising a methacrylic add copolymer and a phthalate polymer.

2. The composition as described in claim 1 wherein the bitter tasting drug is selected from the group consisting of macrolide antibiotics, fluoroquinolones and cephalosporins.

3. The composition as described in claims 1 and 2 wherein the bitter tasting drug is selected from the group consisting of erythromycin, clarithromycin, ciprofloxacin, norfloxacin, cefuroxime, ceftriaxone, chloramphenicol, chloropromazine, and their pharmaceutically acceptable salts and esters.

4. The composition as described in claim 1 wherein the methacrylic acid copolymer is selected from the group consisting of methylmethacrylic ester copolymers and copolymers of ethyl acrylate and methacrylic acid.

5. The composition as described in claim 1 wherein the phthalate polymer is selected from the group consisting of cellulose acetate phthalate, ethylvinyl phthalate, polyvinyl acetate phthalate and hydroxy alkyl cellulose phthalate.

6. The composition as described in claim 1 wherein the ratio of methacrylic acid copolymer to phthalate polymer is between 1:9 to 9:1.

7. The composition as described in claim 1 wherein the combined w/w ratio of the polymers to the drug is at least 1:4.

8. The composition as described in claim 1 wherein the composition is in granular form.

9. The composition of claim 8 wherein the granules include chanelling agents selected from the group consisting of croscarmellose sodium, crospovidone, sodium starch glycolate, lactose, mannitol, sodium chloride, talc, polyvinyl pyrrolidone, carbomer, and xanthan gum.

10. The composition of claim 9 wherein the granules are coated.

11. The composition of claims 9 and 10 wherein the granules are mixed with sugar or artificial sweeteners and/or flavoring agents.

12. The composition of claim 9 wherein the taste masked granules are formulated as dry syrups, suspensions, conventional whole, chewable, dispersible tablets or any other suitable oral dosage form.

13. A taste masked matrix composition comprising a bitter tasting drug, methyl methacrylic ester copolymer, and hydroxypropyl methyl cellulose phthalate.

14. A process for the preparation of a taste masked matrix comprising dissolving the bitter tasting drug, a methacrylic acid copolymer and phthalate polymer in a suitable organic solvent and recovering said taste masked matrix from the solution thereof.

15. The process of claim 14 wherein the dissolving happens in the presence of water.

16. A process as described in claim 14 wherein the bitter tasting drug is chosen from the group consisting of macrolide antibiotics, fluoroquinolones and cephalosporins.

17. The process of claim 14 wherein the bitter tasting drug is selected from the group consisting of erythromycin, clarithromycin, ciprofloxacin, norfloxacin, cefuroxime, ceftriaxone, chlorampheniol, chloropromazine, and their pharmaceutically acceptable salts and esters.

18. The process of claim 14 wherein the methacrylic acid copolymer is selected from the group consisting of methyl methacrylic ester copolymers and copolymers of ethyl acrylate and methacrylic acid.

19. The process of claim 14 wherein the phthalate polymer is selected from the group consisting of cellulose acetate phthalate, ethylvinyl phthalate, polyvinyl acetate phthalate and hydroxy alkyl cellulose phthalate.

20. The process of claim 14 wherein the ratio of methacrylic acid copolymer to phthalate polymer is between 1:9 to 9:1.

21. The process of claim 14 wherein the combined w/w ratio of two polymers to the drug is at least 1:4.

22. The process of claim 14 wherein the organic solvents used are selected from ketones, alcohols, esters or mixtures thereof with or without water.

23. The process of claim 14 wherein the matrix is recovered by a method, selected from the group consisting of evaporation, vacuum evaporation, tray drying, spray drying, drum and belt film drying.

24. The drying process of claim 22, wherein the dried product is compacted to granules.

25. The process of claim 23 wherein the compacted granules are coated.

26. The process of claim 22 wherein the granules are mixed with sugar or artificial sweeteners and/or flavoring agents.

27. The process of claim 14 wherein the taste masked granules are formulated as dry syrups, suspensions, conventional whole, chewable, or dispersible tablets.

28. A process for the preparation of a taste masked matrix comprising a bitter tasting drug, methyl methacrylic ester copolymer and hydroxypropylmethylcellulose phthalate wherein the drug and the two polymers are dissolved in acetone, followed by recovery of the said taste masked matrix from the solution thereof.

29. The process of claim 28 comprising cefuroxime axetil, wherein the matrix is recovered by spray drying.

30. The process of claim 28 comprising clarithromycin.

31. A taste masked matrix composition comprising a bitter tasting drug, and a combination of two polymers comprising a methacrylic acid copolymer and a phthalate polymer, effective to mask the taste, and which releases more than 60% of the drug at a pH 6.8 in one hour.

## Patentansprüche

1. Geschmacksmaskierte Matrixzubereitung, umfassend ein bitter schmeckendes Arzneimittel und eine Kombination von zwei magensaftresistenten Polymeren, umfassend ein Methacrylsäurecopolymer und ein Phthalatpolymer.

2. Zubereitung gemäß Anspruch 1, worin das bitter schmeckende Arzneimittel aus der Gruppe, bestehend aus Makrolid-Antibiotika, Fluorchinolonen und Cephalosporinen, ausgewählt ist.

3. Zubereitung gemäß den Ansprüchen 1 und 2, worin das bitter schmeckende Arzneimittel aus der Gruppe, bestehend aus Erythromycin, Clarithromycin, Ciprofloxacin, Norfloxacin, Cefuroxim, Ceftriaxon, Chloramphenicol, Chlorpromazin und deren pharmazeutisch zulässige Salze und Ester, ausgewählt ist.

4. Zubereitung gemäß Anspruch 1, worin das Methacrylsäurecopolymer aus der Gruppe, bestehend aus Methylmethacrylestercopolymeren und Copolymeren von Ethylacrylat und Methacrylsäure, ausgewählt ist.

5. Zubereitung gemäß Anspruch 1, worin das Phthalatpolymer aus der Gruppe, bestehend aus Celluloseacetatphthalat, Ethylvinylphthalat, Polyvinylacetatphthalat und Hydroxialkylcellulosephthalat, ausgewählt ist.

6. Zubereitung gemäß Anspruch 1, worin das Verhältnis von Methacrylsäurecopolymer zu Phthalatpolymer zwischen 1:9 bis 9:1 beträgt.

7. Zubereitung gemäß Anspruch 1, worin das kombinierte Gewichtsverhältnis von den Polymeren zu dem Arzneimittel mindestens 1:4 beträgt.

8. Zubereitung gemäß Anspruch 1, worin die Zubereitung in einer granulösen Form vorliegt.

9. Zubereitung gemäß Anspruch 8, worin die Granalien Kanal bildende Mittel umfassen, die aus der Gruppe, bestehend aus Croscarmellose Natrium, Crospovidon, Natrium-Stärke-Glycolat, Lactose, Mannit, Natriumchlorid, Talg, Polyvinylpyrrolidon, Carbomer und Xanthan, ausgewählt sind.

10. Zubereitung gemäß Anspruch 9, worin die Granalien beschichtet sind.

11. Zubereitung gemäß den Ansprüche 9 und 10, worin die Granalien mit Zucker oder künstlichen Süßungsmitteln und/oder Geschmacksstoffen vermischt sind.

12. Zubereitung gemäß Anspruch 9, worin die geschmacksmaskierten Granalien als trockene Sirupe, Suspensionen, übliche ganze, kaubare, dispergierbare Tabletten oder jegliche andere geeignete orale Arzneiform zubereitet sind.

13. Geschmacksmaskierte Matrixzubereitung, umfassend ein bitter schmeckendes Arzneimittel, mit Methylmethacrylestercopolymer und Hydroxipropylmethylcellulosephthalat.

14. Verfahren zur Herstellung einer geschmacksmaskierten Matrix, umfassend Auflösen des bitter schmeckenden Arzneimittels, eines Methacrylsäurecopolymers und eines Phthalatpolymers in einem geeigneten organischen Lösungsmittel und Gewinnen der geschmacksmaskierten Matrix aus der Lösung davon.

15. Verfahren gemäß Anspruch 14, worin das Auflösen in Gegenwart von Wasser geschieht.

16. Verfahren gemäß Anspruch 14, worin das bitter schmeckende Arzneimittel aus der Gruppe, bestehend aus Makrolid-Antibiotika, Fluorchinolonen und Cephalosporinen ausgewählt wird.

17. Verfahren gemäß Anspruch 14, worin das bitter schmeckende Arzneimittel aus der Gruppe, bestehend aus Erythromycin, Clarithromycin, Ciprofloxacin, Norfloxacin, Cefuroxim, Ceftriaxon, Chlorampheniol, Chlorpromazin, und deren pharmazeutisch zulässige Salze und Ester, ausgewählt ist.

18. Verfahren gemäß Anspruch 14, worin das Methacrylsäurecopolymer aus der Gruppe, bestehend aus Methylmethacylestercopolymeren und Copolymeren von Ethylacrylat und Methacrylsäure, ausgewählt wird.

19. Verfahren gemäß Anspruch 14, worin das Phthalatpolymer aus der Gruppe, bestehend aus Celluloseacetatphthalat, Ethylvinylphthalat, Polyvinylacetatphthalat und Hydroxialkylcellulosephthalat, ausgewählt wird.

20. Verfahren gemäß Anspruch 14, worin das Verhältnis von Methacrylsäurepolymer zu Phthalatpolymer zwischen 1:9 bis 9:1 beträgt.

21. Verfahren gemäß Anspruch 14, worin das kombinierte Gewichtsverhältnis der zwei Polymere zum Arzneimittel mindestens 1:4 beträgt.

22. Verfahren gemäß Anspruch 14, worin die verwendeten organischen Lösungsmittel aus den Ketonen, Alkoholen, Estern oder Mischungen davon mit oder ohne Wasser ausgewählt sind.

23. Verfahren gemäß Anspruch 14, worin die Matrix durch ein Verfahren gewonnen wird, ausgewählt aus der Gruppe, bestehend aus Verdampfen, Vakuumverdampfen, Hordentrocknen, Sprühtrocknen, Walzen- und Banddünnschichttrocknen (drum and belt film drying).

24. Trocknungsverfahren gemäß Anspruch 22, worin das getrocknete Produkt zu Granalien verdichtet wird.

25. Verfahren gemäß Anspruch 23, worin die verdichteten Granalien beschichtet werden.

26. Verfahren gemäß Anspruch 22, worin die Granalien mit Zucker oder künstlichen Süßungsmitteln und/oder Geschmacksstoffen vermischt werden.

27. Verfahren gemäß Anspruch 14, worin die geschmacksmaskierten Granalien als trockene Sirupe, Suspensionen, übliche ganze, kaubare oder dispergierbare Tabletten zubereitet sind.

28. Verfahren zur Herstellung einer geschmacksmaskierten Matrix, umfassend ein bitter schmeckendes Arzneimittel, Methylmethacrylestercopolymer und Hydroxipropylethylcellulosephthalat, worin das Arzneimittel und die zwei Polymere in Aceton gelöst werden, gefolgt von der Gewinnung der geschmacksmaskierten Matrix aus der Lösung davon.

29. Verfahren gemäß Anspruch 28, umfassend Cefuroxim-Axetil, worin die Matrix durch Sprühtrocknen gewonnen wird.

30. Verfahren gemäß Anspruch 28, umfassend Clarithromycin.

31. Geschmacksmaskierte Matrixzubereitung, umfassend ein bitter schmeckendes Arzneimittel und eine Kombination von zwei Polymeren, umfassend ein Methacrylsäurecopolymer und ein Phthalatpolymer, wirksam zur Maskierung des Geschmacks, und welche mehr als 60 % des Arzneimittels bei einem pH von 6,8 in einer Stunde freisetzt.

## Revendications

1. Composition matricielle à goût masqué comprenant un médicament à goût amer, et une combinaison de deux polymères entériques comprenant un copolymère d'acide méthacrylique et un polymère de phtalate.

2. Composition comme décrite dans la revendication 1, dans laquelle le médicament à goût amer est choisi à partir du groupe composé d'antibiotiques macrolides, de fluoroquinolones et de céphalosporines.

3. Composition comme décrite dans les revendications 1 et 2, dans laquelle le médicament à goût amer est choisi à partir du groupe composé d'érythromycine, clarithromycine, ciprofloxacine, norfloxacine, céfuroxime, ceftriaxone, chloramphénicol, chlorpromazine, et leurs esters et sels pharmaceutiquement acceptables.

4. Composition comme décrite dans la revendication 1, dans laquelle le copolymère d'acide méthacrylique est choisi à partir du groupe. composé de copolymères d'ester méthylméthacrylique et de copolymères d'acrylate d'éthyle et d'acide méthacrylique.

5. Composition comme décrite dans la revendication 1, dans laquelle le polymère de phtalate est choisi à partir du groupe composé d'acétophtalate de cellulose, de phtalate d'éthylvinyle, d'acétophtalate de polyvinyle et de phtalate d'hydroxyalkylcellulose.

6. Composition comme décrite dans la revendication 1, dans laquelle le rapport du copolymère d'acide méthacrylique au polymère de phtalate est entre 1:9 à 9:1.

7. Composition comme décrite dans la revendication 1, dans laquelle le rapport p/p combiné des polymères au médicament est au moins de 1:4.

8. Composition comme décrite dans la revendication 1, dans laquelle la composition est sous forme granulaire.

9. Composition selon la revendication 8, dans laquelle les granules incluent des agents d'acheminement choisis à partir du groupe composé de croscamellose sodium, crospovidone, glycolate d'amidon de sodium, lactose, mannitol, chlorure de sodium, talc, polyvinylpyrrolidone, carbopol et gomme de xanthane.

10. Composition selon la revendication 9, dans laquelle les granules sont enrobés.

11. Composition selon les revendications 9 et 10, dans laquelle les granules sont mélangés avec du sucre ou des édulcorants artificiels et/ou des aromatisants.

12. Composition selon la revendication 9, dans laquelle les granules à goût masqué sont préparés comme sirops anhydres, suspensions, comprimés dispersibles, croquables, entiers usuels ou une quelconque autre formulation orale appropriée.

13. Composition matricielle à goût masqué comprenant un médicament à goût amer, un copolymère d'ester méthylméthacrylique et un phtalate d'hydroxypropylméthycellulose.

14. Procédé pour la préparation d'une matrice à goût masqué consistant à dissoudre le médicament à goût amer, un copolymère d'acide méthacrylique et un polymère de phtalate dans un solvant organique approprié et à récupérer ladite matrice à goût masqué à partir de sa solution.

15. Procédé selon la revendication 14, dans lequel la dissolution se produit en présence d'eau.

16. Procédé comme décrit dans la revendication 14, dans lequel le médicament à goût amer est choisi à partir du groupe composé d'antibiotiques macrolides, de fluoroquinolones et de céphalosporines.

17. Procédé selon la revendication 14, dans lequel le médicament à goût amer est choisi à partir du groupe composé d'érythromycine, clarithromycine, ciprofloxacine, norfloxacine, céfuroxime, ceftriaxone, chloramphénicol, chlorpromazine, et leurs esters et sels pharmaceutiquement acceptables.

18. Procédé selon la revendication 14, dans lequel le copolymère d'acide méthacrylique est choisi à partir du groupe composé de copolymères d'ester méthylméthacrylique et de copolymères d'acrylate d'éthyle et d'acide méthacrylique.

19. Procédé selon la revendication 14, dans lequel le polymère de phtalate est choisi à partir du groupe composé d'acétophtalate de cellulose, de phtalate d'éthylvinyle, d'acétophtalate de polyvinyle et de phtalate d'hydroxyalkylcellulose.

20. Procédé selon la revendication 14, dans lequel le rapport du copolymère d'acide méthacrylique au polymère de phtalate est entre 1:9 à 9:1.

21. Procédé selon la revendication 14, dans lequel le rapport p/p combiné des deux polymères au médicament est au moins de 1:4.

22. Procédé selon la revendication 14, dans lequel les solvants organiques utilisés sont choisis à partir de cétones, alcools, esters ou mélanges de ceux-ci avec ou sans eau.

23. Procédé selon la revendication 14, dans lequel la matrice est récupérée par un processus, choisi à partir du groupe comprenant l'évaporation, l'évaporation sous vide, le séchage sur plateau, le séchage par atomisation, le séchage sur bande et sur tambour.

24. Procédé de séchage selon la revendication 23, dans lequel le produit séché est compressé en granules.

25. Procédé selon la revendication 23, dans lequel les granules compressés sont enrobés.

26. Procédé selon la revendication 22, dans lequel les granules sont mélangés avec du sucre ou des édulcorants artificiels et/ou des aromatisants.

27. Procédé selon la revendication 14, dans lequel les granules à goût masqué sont préparés comme sirops anhydres, suspensions, comprimés entiers usuels, croquables ou dispersibles.

28. Procédé pour la préparation d'une matrice à goût masqué comprenant un médicament à goût amer, un copolymère d'ester méthylméthacrylique et un phtalate d'hydroxypropylméthycellulose, où le médicament et les deux polymères sont dissous dans de l'acétone, suivi par la récupération de ladite matrice à goût masqué à partir de sa solution.

29. Procédé selon la revendication 28, comprenant du céfuroxime axetil, dans lequel la matrice est récupérée par séchage par atomisation.

30. Procédé selon la revendication 28, comprenant de la clarithromycine.

31. Composition matricielle à goût masqué comprenant un médicament à goût amer et une combinaison de deux polymères comprenant un copolymère d'acide méthacrylique et un polymère de phtalate, efficace pour masquer le goût et qui libère plus de 60% du médicament à un pH 6,8 en une heure.
